# EUROPEAN PATENT APPLICATION

(11) **EP 1 894 913 A1**
(43) Date of publication of application: **05.03.2008**
(21) Application number: 06017333.3
(22) Date of filing: 21.08.2006
(51) Int. Cl.: C07C 67/03, C11C 1/10, C11C 3/00, C07C 69/24, C07C 69/52

(54) **Production of esters of fatty acids and lower alcohols**

(71) Applicant: Desmet Ballestra Oleo s.p.a., 00040 Pomezia RM (IT)
(72) Inventor: Adami, Icilio, 20049 Concorezzo (Milano) (IT); Soragna, Francesco, 00128 - Roma (IT); Kellens, Marc, 2812 Mechelen-Muizen (BE)
(74) Representative: Bird, William Edward

(57) **Abstract**

The present invention provides a process for the production of esters of fatty acids and C₁-C₅ alkyl alcohols comprising the steps of:
(e) neutralising a fatty feed comprising a triglyceride oil or fat, partial glycerides and/or free fatty acids by vacuum stripping at a temperature from 200°C to 280°C, thus providing a vapour stream and a residue,
(f) collecting a distillate by scrubbing said vapour stream,
(g) transesterifying said residue with a C₁-C₅ alkyl alcohol while using an alkaline catalyst,
(h) separating the transesterification reaction mixture from step (c) into a fraction comprising C₁-C₅ alkyl esters of fatty acids and an alcoholic fraction.

## Description

The invention relates to a process for the production of fatty acid esters of lower alkyl alcohols which can be used as a fuel in compression ignition ('diesel') engines.

### BACKGROUND OF THE INVENTION

Lower alcohol esters of fatty acids have many applications. They are for instance used as intermediates in the production of fatty alcohols and other oleo-chemicals and more recently, they have also been used as fuel for compression ignition engines. Consequently, several processes for the production of lower alcohol, and especially methanol, esters of fatty acids have been developed to cope with a large variety of raw materials ranging from fully refined oils to greases with a high free fatty acid content.

Originally, fatty acid esters and in particular fatty acid methyl esters (FAME) have been produced industrially as an intermediate product in soap making. Accordingly, a process is known comprising reacting a higher fatty acid glyceride with a saturated aliphatic monohydric alcohol having less than 5 carbon atoms in the presence of a small amount of an alkali metal hydroxide under substantially anhydrous conditions. This alcoholysis or transesterification process leads to the formation of a fatty acid ester phase and a glycerol phase which also contains the soaps formed during the process. In order to minimise the amount of catalyst required and the amount of soap formed, the oil should be dry and substantially free from free fatty acids (FFA) and mucilaginous vegetable matter.

Although subsequently there have been used raw materials with appreciable amounts of FFA, the presence of FFA is nevertheless considered to be a disadvantage not only because of the increased use of alkaline catalyst but especially because the formation of a gel which prevents or slows down separation and settling of the glycerol formed during the transesterification. Accordingly, J. Van Gerpen et al. in Biodiesel Production Technology (July 2004) devote an entire chapter to the pre-treatment of high free fatty acid feedstocks which lists four methods. The first method mentioned uses enzymes such as lipases The second method mentioned is concerned with glycerolysis. It involves adding glycerol to the feedstock and heating it to high temperature so that the water formed by the esterification of the FFA with the glycerol evaporates and shifts the equilibrium towards the partial glyceride side. Thus, said free fatty acids in the stock are substantially completely esterified, so that the reaction mixture can then be subjected to alcoholysis by reacting with a low molecular weight monohydric alcohol to form low molecular weight monohydric alcohol esters of fatty acids. The drawbacks of the glycerolysis reaction are a high temperature and the fact that it is relatively slow.

The third method mentioned involves the use of an acid catalyst to catalyse both the esterification of the free fatty acids with a lower alcohol and the transesterification of the glycerides. This acid catalysed transesterification is very slow, taking several days to complete, and the water formed during the esterification ultimately stops the reaction.

A fourth method comprises an acid catalysed esterification followed by an alkali catalysed transesterification, an approach which solves the reaction rate problem by using each technique to accomplish the process for which it is best suited. Accordingly, a process is known which comprises treating a fatty acid ester of a higher alcohol (e.g. a glyceride) containing free fatty acids with an alcohol in the presence of an acidic esterification catalyst, and thereafter reacting said fatty ester of a higher alcohol with an aliphatic monohydric alcohol having one to six carbon atoms per molecule in the presence of sufficient alkaline agent to neutralize any acid present and to provide an alkaline alcoholysis catalyst.

An improved process employs so much alcohol during the esterification step that it forms a separate alcohol phase containing most of the acid esterification catalyst. After the esterification reaction, the phases are separated from each other, and consequently, less alkali is required for the neutralisation of the acid catalyst present in the fatty phase than without this separation process. Another process also uses so much alcohol that a separate phase is formed and it also comprises washing the fatty phase with a mixture of glycerol and methanol to remove residual water and acid catalyst. This reduces the amount of alkali required for the neutralisation of the acid esterification catalyst even further.

In addition to methods for dealing with high acidity oils, the following may also be listed: alkali refining, solvent extraction, steam refining and the use of an excess of alkali. A common disadvantage inherent to these four additional methods is that they decrease the yield because the FFA present in the feedstock is not converted to fatty acid esters of lower alcohols. The specific disadvantage of the alkali refining process is the by-product soapstock which requires special treatment. Solvent extraction methods imply the use of large volumes of solvent which is costly both with respect to investment and running costs. Steam refining suffers from both disadvantages mentioned for the previous methods in that it requires relatively large equipment and also involves the problem of treating the distillates, and finally, the use of excess caustic to neutralise the FFA present in the feed leads to emulsion formation, poor phase separation and loss of materials.

Accordingly, there is full agreement that esterification of the FFA present in the fatty feed followed by transesterification of the esterification reaction mixture constitutes the optimal way to process fatty feeds with FFA. With respect to the acid catalysts to be used during the esterification step, prior art describes the use of sodium bisulphateor the use of a strongly acid ion exchange resin, both of which catalysts have the advantage that they can be removed from the reaction mixture by filtration.

Although the esterification of the FFA present in the fatty feed reduces the amount of soaps formed by the alkaline catalyst, it does not totally suppress soap formation since the alkali used to catalyse the transesterification eventually reacts to form soap since FAME tend to saponify much faster than triglycerides. During the transesterification, this soap will concentrate in the glycerol layer and acidulation of the glycerol phase will cause the separation of a fatty stream that is rich in FFA. If not reintroduced into the process, this stream will constitute a loss. Accordingly, it is known to separate this fatty stream from glycerol, to esterify this fatty stream with an alcohol while using an acid catalyst, and to recycle the fatty acid esters thus formed into the stream to be transesterified. It is also known to separate this fatty stream from glycerol but, instead of esterifying the fatty stream separately, to mix the stream with the raw material to be transesterified, and then esterify the mixture.

Another method to recycle the FFA-rich fatty stream resulting from the acidulation of the glycerol phase is known. By transforming these fatty substances into glycerides, preferably triglycerides, they can be added to the starting material and thereby recycled. The use of an alkaline catalyst allows much higher temperatures to be used than when an acid catalyst were to be used, so that the water formed by the esterification, being more volatile than glycerol, can be distilled off causing the esterification equilibrium to be shifted to the glyceride side. High temperature and alkaline catalysed esterification with glycerol followed by methanolysis is also known.

Although the prior art methods described above effectively aim at maximising the fatty acid ester yield, they also have disadvantages. Esterifying a mixture of glycerides and FFA requires much larger vessels and more energy for heating and cooling than when the FFA were concentrated. This is especially serious since the esterification process is rather slow. Moreover, the prior art methods hardly remove the impurities present in the raw material and this has two quite serious consequences. Lipophilic impurities will therefore concentrate in the fatty ester phase and thereby cause the properties of the final product to vary in an unpredictable manner.

Moreover, impurities will affect the performance of the process. They may increase catalyst requirements, cause emulsions to be formed and thus slow down or inhibit phase separation processes, and again the effect may be unpredictable. These effects will of course be less serious if the raw material is highly purified but this adds to the costs and prohibits taking advantage of opportunities to purchase and process low-grade and cheap raw materials such as spent deep frying oil or trap greases.

### OBJECTS AND ADVANTAGES OF THE INVENTION

Accordingly, it is an object of the invention to overcome the various disadvantages of the prior art processes and systems for making esters of fatty acids and lower alkyl alcohols.

An advantage of the invention to eliminate harmful impurities from the reaction system of processes for making esters of fatty acids and lower alkyl alcohols

It is also an advantage of the invention to provide a process for making esters of fatty acids and lower alkyl alcohols which is able to process a wide range of fatty feed raw materials.

It is a further advantage of the invention to incorporate FFA-rich streams into the fatty feed raw material to be converted into fatty acid esters, and thus to constitute an outlet for waste or by-product streams comprising fatty acid moieties.

It is also an advantage of the invention to maximise the yield of production of fatty acid esters.

It is yet another advantage of the invention to minimise the number and/or the size of the reaction vessels required for performing the production of esters of fatty acids and lower alkyl alcohols.

It is a further advantage of the invention to reduce the amount of catalyst used in the production of esters of fatty acids and lower alkyl alcohols.

Further objects and advantages of the invention will become apparent from the description and the examples hereinafter.

### SUMMARY OF THE INVENTION

It has surprisingly been found that most of the above objects can be attained by applying the transesterification process producing fatty acid esters of lower alcohols to a fatty feed raw material that has first been subjected to a vacuum stripping process and, optionally, prior to said vacuum stripping step, to an acid refining or dry degumming step. Apparently, although the inventors intention is not to be bound by theory, this vacuum stripping process removes some raw material constituents either by thermal breakdown or by volatilisation. Accordingly, the process according to the invention comprises the steps of neutralising a fatty feed raw material by vacuum stripping at an elevated temperature, then transesterifying this neutralised material, and finally isolating the fatty acid esters of lower alcohols from the transesterification reaction mixture.

The present invention also provides process equipment for the production of esters of fatty acids and C₁-C₅ alkyl alcohols comprising:
(a) Means for neutralising a fatty feed comprising a triglyceride oil or fat, partial glycerides and/or free fatty acids by vacuum stripping at a temperature from 200°C to 280°C, thus providing a vapour stream and a residue,
(b) Means for collecting a distillate by scrubbing said vapour stream,
(c) Means for transesterifying said residue with a C₁-C₅ alkyl alcohol while using an alkaline catalyst, and
(d) Means for separating the transesterification reaction mixture into a fraction comprising C₁-C₅ alkyl esters of fatty acids and an alcoholic fraction.

Additional embodiments of the process according to the invention comprise means to increase the fatty acid ester yield by esterifying free fatty acids as removed during the vacuum stripping process and formed during the transesterification. By only esterifying high FFA content substrates, the size of the required esterification equipment is significantly minimised.

### BRIEF DESCRIPTION OF THE DRAWINGS

The present invention is illustrated by the embodiments shown in the appended drawings, in which:
FIG. 1 represents a flow diagram incorporating the high temperature vacuum stripping of the raw material, the transesterification of the neutralised raw material and the after-treatment of both phases of the transesterification reaction mixture.
FIG. 2 represents flow diagrams of various processes leading to FFA-rich by-products.
FIG. 3 represents a flow diagram showing the esterification process of FFA-rich streams with a lower monohydric alcohol and the recycling of the esterification product into the process according to the invention.
FIG. 4 represents a flow diagram showing the esterification process of FFA-rich streams with glycerol and the recycling of the esterification product into the process according to the invention.

### DEFINITION OF TERMS

The terms to be defined below are shown in capitals and listed alphabetically, and if a definition contains a listed term, this is shown by the italicisation of this term.
• ACID OILS is the fatty product that results from the *acidulation* of *soapstock.* Its composition varies but it is likely to contain more than 50 % by weight of free fatty acids, the remainder comprising triglyceride oil, *partial glycerides* and unsaponifiables.
• AICD REFINING is a *degumming* process comprising the treatment of the oil to be degummed with a degumming acid such as phosphoric acid or citric acid, which in a second stage, is partially neutralised causing the gums to hydrate so that they can be removed as a separate phase.
• ACIDULATION is the process used to recover the fatty matter contained in product streams comprising soaps. It involves adding an acid as for instance sulphuric acid, to this product stream and separating the fatty phase as *acid oils* from the aqueous phase.
• ALCOHOLYSIS is the reaction between an alcohol and a glyceride such as an oil or fat. If the alcohol concerned is methanol, the *alcoholysis* can also be referred to as 'methanolysis' and if it is glycerol, the term 'glycerolysis' can be used; alcoholysis is also referred to as *transesterification.*
• ALKALI REFINING is the treatment of a *crude oil* with alkali to remove the free fatty acids *(FFA)* and phosphatides present in the *crude oil* as a *soapstock.* The treatment is also referred to as 'chemical refining'
• CRUDE OIL is the general name for an oil or fat as isolated from its source and that has not undergone any treatment except perhaps a water *degumming* treatment ensuring that the crude oil meets trading specifications and does not throw a deposit during storage and transport. Crude oil therefore may contain free fatty acids and/or gums.
• DEGUMMING is the general term for the removal of phosphatides and other mucilaginous matter from *crude oil.* The water-degumming process only removes hydratable phosphatides and thus leaves the non-hydratable phosphates (NHP) in the water-degummed oil. NHP removal necessitates the use of a degumming acid.
• ESTERIFICATION is the formation of an ester from an alcohol and an acid such as a fatty acid, whereby water is formed at the same time. Usually, an acid catalyst such as sulphuric acid is used but enzymatic catalysts like lipases can also be effective in increasing the rate of reaction.
• FAME is the abbreviation of Fatty Acid Methyl Esters.
• FATTY FEED is a general description of a raw material comprising triglyceride oil, partial glycerides and/or *FFA.*
• FFA is the standard abbreviation of Free Fatty Acids.
• HYDROLYSIS is the reverse reaction of the *esterification* and in practice these reactions lead to equilibria. A high degree of hydrolysis therefore results from increasing the water concentration, whereas low concentrations of free acids and/or free alcohols can be induced by water removal.
• INTERESTERIFICATION is the reaction between different triglycerides that results in an often statistical redistribution of the fatty acid moieties over the glycerol moieties. The reaction is catalysed by a base or lipase enzyme. In the context of the process according to the present invention, interesterification is hardly relevant but the term has been defined to avoid confusion.
• NEUTRALISATION is the removal of free fatty acids from a *crude* or *degummed oil.*
• PARTIAL GLYCERIDES are partially hydrolysed triglycerides. Diacyl glycerol, having two acyl groups and one free hydroxyl group is commonly referred to as a diglyceride. A monoglyceride has only one acyl group left and thus has two free hydroxyl groups.
• PHYSICAL REFINING is the *neutralisation* process whereby the free fatty acids are removed by a vacuum stripping process.
• REFINING is a general term applied to the various steps that convert a crude oil into a consumer product and thus entails steps such as *degumming, neutralisation,* bleaching and deodorisation. In this application the term refining is not limited to the *neutralisation* step. 'Chemical refining' or "alkali refining", and 'physical refining' refer to aspects of the generic concept of refining.
• SAPONIFICATION is the splitting of a fatty acid ester bond by alkali and results in the formation of soaps.
TRANSESTERIFICATION is another name for *alcoholysis.*

### DETAILED DESCRIPTION OF THE INVENTION

The present invention will be described with respect to particular embodiments and with reference to the drawings but the invention is not limited thereto but only by the claims.

The process for making esters of fatty acids and lower alkyl alcohols according to the invention has in common with various prior art processes that it uses a neutral fatty feed as a feedstock for the transesterification step but a significant distinctions is the use of a neutral fatty feed that originates from a vacuum stripping process.

The type of triglyceride oil or fat to be used in the feed of the process according to the invention is not critical. It can be a vegetable oil such as, but not limited to, rapeseed oil, soybean oil, palm oil or coconut oil, and it can also be an animal fat such as, but not limited to, tallow. It can also be a waste product such as, but not limited to, spent deep frying oil or trap grease. Thus one object of the invention to allow a wide range of raw materials including cheap waste or by-products to be converted into lower alkyl alcohol esters is hereby met. For practical purposes, it may be advantageous to blend widely different raw materials so as to supply the process according to the invention with a less variable feed. Blending criteria can also be prescribed by final product specifications.

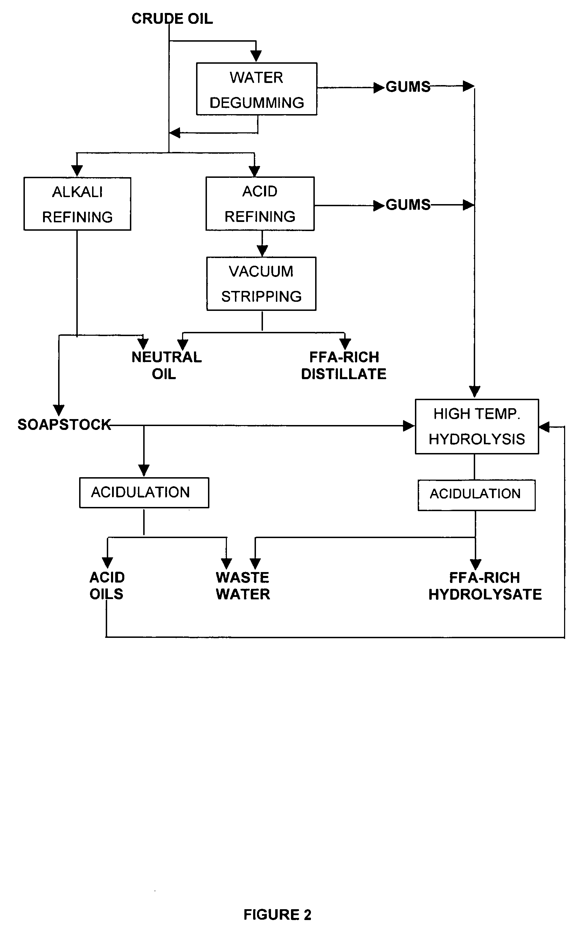

As shown in FIG. 2, the crude oil may be water degummed and/or acid refined prior to the vacuum stripping treatment. According to the invention, it is not necessary to bleach the crude oil before it is stripped under vacuum. The optional and preferred omission of the bleaching step constitutes a substantial saving and environmental advantage over some prior art processes in that it obviates the purchase of bleaching earth, the oil loss by entrainment in spent earth, and the disposal of the spent earth.

The crude oil or fat may be water degummed, or the oil or fat purchased as raw material for the process according to the invention may already have been water degummed after having been expelled and/or extracted from its raw material to meet a purchase specification and/or to prevent a deposit from being thrown during transport and/or storage. This water degumming process may have lowered the phosphatide level in the crude oil to, preferably, less than about 25 ppm phosphorus, more preferably less than about 10 ppm. In that instance, a further degumming process such as dry degumming or acid refining is not imperative, but if the phosphorus content exceeds one of the above-mentioned levels, then a degumming treatment is recommended.

A degumming process that has been found to be highly effective in lowering the phosphorus content of the crude oil below about 25 ppm is the so-called acid refining process. This process comprises the dispersing of a degumming acid such as, but not limited to, phosphoric acid in the crude oil, its partial neutralisation with alkali, and the removal of the gum phase by centrifugation. In the process according to the invention, this acid refined oil is then subjected to a high temperature, vacuum stripping treatment.

Another degumming process that has been found to be suitable is the dry degumming treatment. This treatment comprises a heat treatment in the presence of phosphoric acid and a bleaching adsorbent such as bleaching earth. High temperatures will normally be avoided when processing food grade products because they can lead to the formation of conjugated polyunsaturated fatty acids and *trans* isomers. For non-food uses, these isomerisation reactions do not matter and the high temperature treatment has the advantage of being more effective within a shorter period of time, so that less bleaching earth is required. A dry degumming treatment can be followed by a high temperature vacuum stripping treatment after the clay has been removed. This stripping process yields the neutral oil that can be transesterified by the process according to the invention.

This vacuum stripping process, step (a) of the process according to the invention, is preferably executed in a continuous deodoriser as commonly used for the deodorisation of edible oils. The continuous type is preferred over the batch type because it commonly incorporates economisers and thus requires less energy for heating the oil than the batch equipment. Besides, some mixing between subsequent lots of feedstock can be tolerated when producing fatty acid esters for diesel fuel application. However, care should be taken to prevent small amounts of lauric oils like coconut oil or palm kernel oil from mixing with longer chain oils like soybean oil, since for some unknown reason, such mixtures tend to foam.

Although the equipment used for the neutralisation of the raw material to be converted into lower alkyl esters of fatty acids is the same as the equipment used for the deodorisation or steam refining of edible oils, the process conditions are not necessarily the same. Edible oils should meet a specification for final acidity within a range of about 0.03-0.05 % (expressed as oleic acid) and should have a *trans* isomer content below about 1% for soybean oil and canola and below about 0.5% for other oils. In practice, these *trans* isomer specifications limit the stripping temperature to about 250°C or even less. The specification of the fatty feed to be transesterified is less stringent. Its *trans* content has not been directly specified and a residual FFA of about 0.1 % or even 0.2%by weight may be acceptable. Consequently, the feed can be neutralised at temperatures above 260°C and less extensively than food-grade material. Both differences allow the steam usage per tonne to be reduced and throughput to be increased both of which factors independently reduce cost in comparison with deodorising food-grade material.

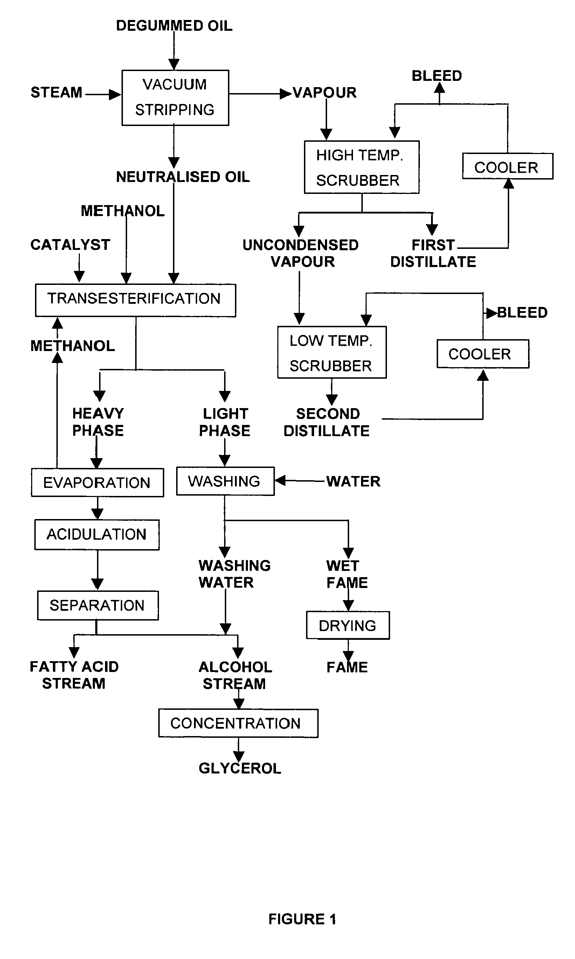

The next step (b) of the process according of the invention is concerned with the collection of the distillate by scrubbing the vapour stream. A preferred embodiment of this step (b) is shown in FIG. 1. It comprises two scrubbers operating at different temperatures to realise a fractional condensation of the vapours leaving the deodoriser in accordance with equipment such as described in U.S. Patent 6,953,499. In the first, high temperature scrubber, the least volatile components of the vapour stream will condense onto the distillate stream circulating over this scrubber. If the temperature of this stream is controlled by the cooler shown in FIG. 1 at around 140°C, fatty acids will hardly condense so that the first distillate stream will be relatively rich in tocopherols and plant sterols. It is collected through the bleed into intermediate storage for outside sale and/or tocopherol and sterol recovery. The first distillate stream also constitutes a purge for minor raw material constituents.

In the embodiment concerned, the vapour leaving the first, high temperature scrubber is then passed to a second scrubber that operates at a lower temperature such as for instance around 50°C, i.e. just above the melting point of fatty acids. As indicated in FIG. 1, this leads to a second distillate, which is also collected through a bleed line. The same distillate is referred to as " FFA-rich distillate " in FIG. 2. As will be described in detail below, this FFA-rich distillate can also be recycled into the process according to the invention and thus increase the fatty ester yield.

The next step (c) of the process according to the invention entails the transesterification of the neutral oil with a lower alkyl alcohol while using an alkaline catalyst. Preferably, the transesterification with the lower alkyl alcohol is with one or more C₁ to C₅, more preferably C₁ to C₃ alcohols, especially methanol. The alkaline catalyst may be, amongst others, a hydroxide like sodium hydroxide or potassium hydroxide, or preferably an alkylate such as, but not limited to, sodium methylate. The alkaline catalyst can be added to the transesterification mixture as 100% solids but preferably as a solution in a suitable solvent such as, but not limited to, anhydrous alcohol, e.g. methanol.

The amount of alcohol to be used in the transesterification step (c) of the process according to the invention may be preferably between about 1.2 and 2.0, more preferably between 1.4 and 1.6, molar equivalents of the fatty acid moieties present in the neutralised oil. The amount of the alkaline catalyst is preferably at most 0.5 % by weight of the neutralised oil. The temperature used in the transesterification step (c) is preferably in the range of about 45-60°C.

Methanol containing the alkaline catalyst is preferably added in stages, such as for instance three stages. Consequently, the substrate is allowed to react with a portion of the methanol containing the alkaline catalyst in a first reactor. The reaction mixture leaving this reactor is then separated into a heavy glycerol layer and a lighter fatty layer, which is then allowed to react with a second portion of the methanol containing the alkaline catalyst, in a second reactor. The heavy phase of the reaction mixture leaving the second reactor is then combined with the heavy phase originating from the first reactor and the light phase is allowed to react with the third portion of methanol containing the transesterification catalyst in the third reactor.

The reaction mixture entering this third reactor will already have a high FAME content. Consequently, the amount of glycerol to be liberated is small and the high concentration of methanol provided by the third portion ensures that the equilibrium is shifted towards almost complete conversion into FAME. The reaction mixture leaving the third reactor is again separated into two phases in step (d) of the process according to the invention but this time, the alcoholic phase is not combined with the heavy phases originating from the first and second reactors but reused as such as methanol with catalyst and fed to the first reactor. Therefore, although the molar ratio of methanol to fatty acid moieties fed to the reactors is well in excess of stoichiometry, at for instance 1.5-2.0, the amount of methanol to be rectified is much smaller than indicated by this ratio.

As indicated in FIG. 1, the FAME stream obtained during the separation step (d) of the process according to the invention can be washed with water to remove the last traces of glycerol. Acidifying this washing water has been found to be advantageous in that it greatly reduces the tendency to form emulsions, and thus facilitates phase separation. When citric acid is used as the water acidifying means, a strength of about 2% by weight has been found to be sufficient. The washing water can be combined with other glycerol streams that are processed to recover their glycerine content. Subsequently, the FAME phase has to be exposed to vacuum to fully dry the product.

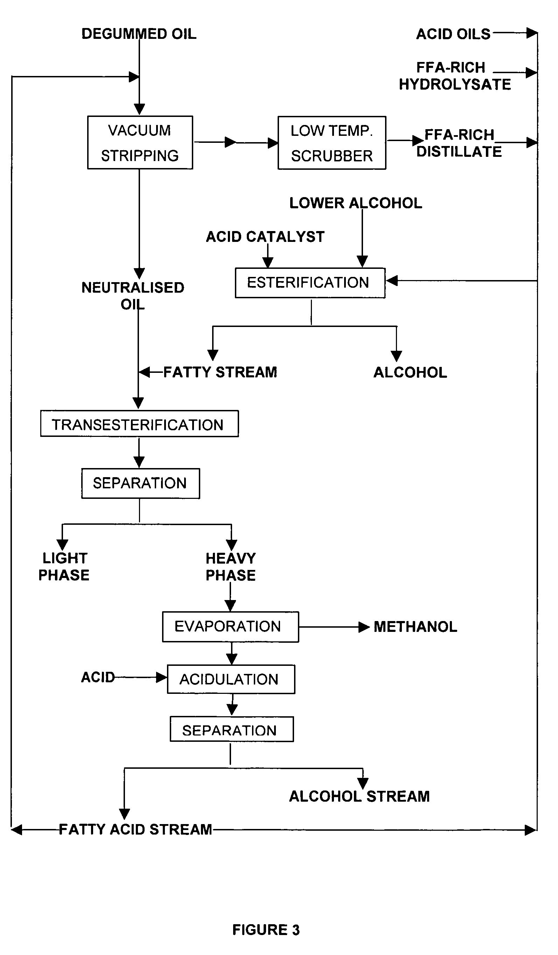

The incorporation of FFA-rich streams into the raw material to be converted into fatty acid esters and the provision of an outlet for waste or by-product streams comprising fatty acid moieties are further objects of the invention. The esterification of these fatty acid moieties achieving these objects is illustrated in FIG. 3 and the origin and generation of such streams is illustrated in FIG. 2. This latter figure shows that the water degumming process of crude oils generates gums. Occasionally, these gums can be dried and sold as lecithinbut a wider and more profitable outlet would be welcome.

This is especially true for the gums originating from the acid refining process since their often high salt content may prohibit these gums to be mixed into the meal. As shown in FIG. 2, the fatty acid moieties present in these gums can be recuperated by a high-temperature hydrolysis process as described by Naudet et al in Revue Française des Corps Gras (1955) 2 (4) 222-224. In this process, the soapstock is heated for a period of about 1-2 hours at some 200°C in equipment that can withstand 20 bar pressure, which treatment causes all ester bonds in the triglycerides and the phosphatides to be hydrolysed. Subsequent acidulation leads to an immediate and sharp separation into an aqueous layer and a fatty layer of acid oils with an FFA content of close to 100%. The separation occurs at a higher pH than needed for soapstock acidulation so less acid is required and waste water disposal is alleviated. Operation at a higher temperature will shorten the process without affecting the quality of the fatty acids.

Apart from recuperating the fatty acid moieties present in waste products and allowing them to be converted into lower alkyl alcohol esters, the above-mentioned high temperature hydrolysis process may also have the advantage of inactivating compounds that would otherwise adversely affect performance of the process, for instance when these compounds can act as emulsifiers and delay or inhibit phase separations. Accordingly, a preferred embodiment of the process according to the invention comprises the high temperature hydrolysis of soapstock originating from the alkali refining process, which has also been illustrated in FIG. 2. The same figure also illustrates the high temperature hydrolysis of the acid oils obtained by the acidulation of soapstock. This embodiment is particularly applicable to soapstock purchased externally and therefore often of dubious quality.

The flow diagram in FIG. 3 shows that the acid oils, the FFA-rich hydrolysate and/or the FFA-rich distillate supplied by the scrubber bleed shown in FIG. 1 are fed to an esterification reactor that is also provided with a lower alkyl alcohol and an acid catalyst. In addition, FIG. 3 also shows that the fatty acid stream originating from the acidulation of the glycerol stream resulting from the transesterification process can also be fed to said esterification reactor. However, the esterification of said fatty acid stream and its subsequent transesterification suffers from the potential disadvantage that impurities present in said fatty acid stream will enter said transesterification reactor or reactors and may impair the separation processes and/or the activity of the alkaline catalyst. Accordingly, a preferred embodiment of the process according to the invention comprises recycling of said fatty acid stream to the degummed oil prior to this oil being deacidified by vacuum stripping. This has the advantage that the FFA present in said fatty acid stream are recuperated in the low temperature scrubber and that other, less desirable constituents can be purged out of the system.

The esterification process is preferably carried out at atmospheric pressure and close to the boiling point of the reaction mixture but other process conditions also fall within the scope of the process according to the invention. A faster esterification is for instance observed when operating at about 100-130°C and a pressure of 7-9 bar. Acid catalysts like sulphuric acid, or sulphonic acids or any other catalysts known to those skilled in the art, may be used as an esterification catalyst. These catalysts are quite effective but nevertheless, it may take quite some time and especially a substantial concentration of catalyst to attain a high conversion and thus a low residual FFA content. After all, the esterification reaction is reversible and the water formed during ester formation affects the position of the equilibrium.

So if the amount of FFA to be esterified is large in comparison with the amount of neutralised oil into which the esterification reaction mixture is to be mixed and/or the residual FFA content after esterification is appreciable, the FFA content of the transesterification mixture may increase to more than about 0.2% by weight. This will in turn increase the amount of alkaline transesterification catalyst required, lead to more soap in the heavy, glycerol phase and a larger fatty acid stream originating from this glycerol phase.

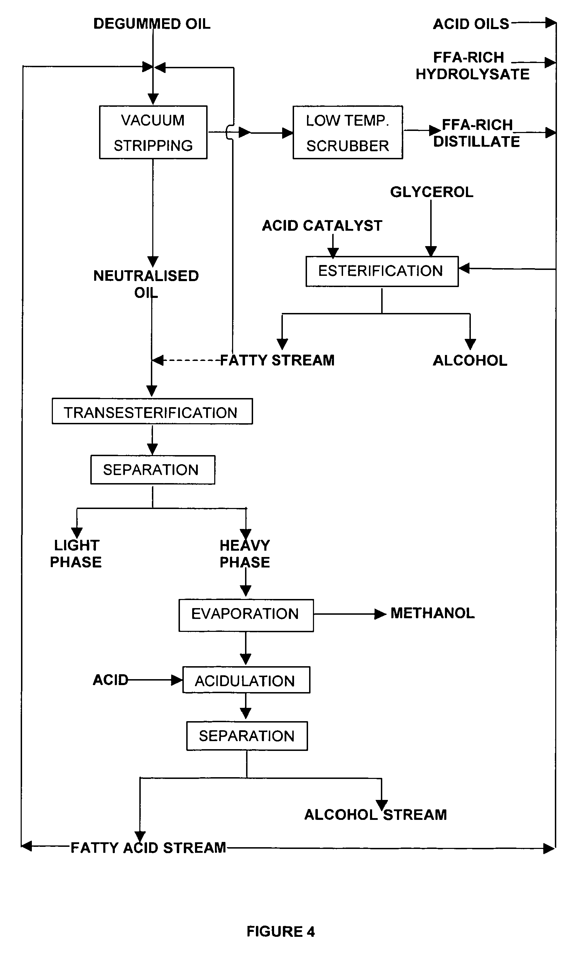

Accordingly, the flow diagram represented in FIG. 4 constitutes another useful embodiment of the process according to the invention. In this embodiment, the alcohol used in the esterification process is not a monohydric alcohol but a polyhydric alcohol like glycerol. The use of glycerol has two clear advantages over the use of a lower alkyl alcohol during the esterification step. The first advantage is that the water formed as a result of the ester formation can be removed by distillation without simultaneous removal of the glycerol because of their large difference in volatility.

The second advantage is that the esterification with glycerol leads to glycerides including partial glycerides, which are considerably less volatile than FFA and FAME. Subjecting the esterification product to a vacuum stripping treatment will therefore cause the removal of the residual, non-esterified FFA, but will not cause the partial glycerides to be volatilised. After vacuum stripping, the residue will therefore have a low to negligible FFA content and will not require extra transesterification catalyst. Moreover, the embodiment depicted in FIG. 4 has the advantage that undesirable constituents in the acid oils and/or the FFA-rich hydrolysate used as starting materials in the esterification process may be removed from the system by the vacuum stripping process.

If the residual FFA-content after the esterification with glycerol is sufficiently low and there is little need to purify the esterification product by high temperature vacuum stripping, the esterification product can also be transesterified directly. This embodiment has been indicated in FIG. 4 by a dotted line. Esterification with glycerol hardly affects the glycerol yield of the process according to the invention since the glycerol bound in the esterification will be liberated in the transesterification process.

Like the esterification with lower alkyl alcohols, esterification with glycerol preferably employs an acidic catalyst such as, but not limited to, sulphuric acid. However, esterification with glycerol can be carried out at a higher temperature, which increases the rate of reaction. A too high temperature should be avoided since this may lead to side reactions. Consequently, a temperature of around 130°C and operation at reduced pressure to selectively evaporate the water formed by the esterification are preferred process conditions.

At the end of both esterification processes, the reaction mixture is split into a fatty stream and an alcohol stream, which alcohol stream will contain most of the acidic catalyst. Consequently, the fatty stream contains so little acid catalyst that it does not require any neutralisation before it is mixed with either the degummed oil or the neutralised oil. If so desired, the fatty stream can be water washed.

The following examples are provided only for the purpose of illustration of the invention and should not be understood as limiting the invention in any aspect.

### EXAMPLE 1

This example illustrates the preparation of a raw material by the acid refining process followed by the steam refining of the acid refined material. The raw material concerned is jatropha oil obtained in India from expelling the toxic physic nut (*Jatropha curcas*). Its fatty acid composition (by weight) was determined as comprising the following: palmitic acid, 16.0%; palmitoleic acid, 1,1%; stearic acid, 6.1 %; oleic acid, 36.5%; linoleic acid, 39.9%; linolenic acid 0.2%. This composition makes the oil highly suitable for biodiesel.

The sample of jatropha oil used in this example had a FFA content of 4.7% (expressed as oleic acid) and contained 60.6 ppm phosphorus, 44.6 ppm iron, 36.4 ppm calcium and 21.4 ppm magnesium, as determined by inductively coupled plasma (ICP) spectroscopy. The citric acid refining process applied to the sample comprises the following steps:
• The oil is heated to 75°C;
• An amount of 0.38% (w/w) of a 30% (w/w) solution of citric acid is added to the oil and dispersed very finely by mixing with a high shear mixer such as an Ultra Turrax® running at 16 000 rpm for 1 minute;
• The acid is allowed to react for 3 minutes while the mixture is agitated gently to prevent the acid from settling;
• Then an amount of 1.23% (w/w) of an 8% (w/w) caustic soda (NaOH) solution is mixed into the acidified oil by using a high shear mixer for 1 minute;
• An amount of 1.5% (w/w) water is added and dispersed by using the high shear mixer at 16,000 rpm for 1 minute;
• The neutralised reaction mixture is allowed to cool to 40°C while being gently agitated over a period of 100 minutes;
• The oil is heated to 70°C to facilitate subsequent separation;
• The heated oil is centrifuged for 15 minutes at 2,000 g to obtain a separation between the acid refined oil and the gums.
The citric acid refining process reduced the phosphorus content from 60.6 ppm to 23.2 ppm. The iron, calcium and magnesium contents were reduced to 13.5, 18.9 and 3.9 ppm respectively. The insoluble impurities which amounted to 20 ppm in the crude oil had also been reduced to negligible amounts by the acid refining process.

The oil was subjected to a batch steam stripping process at a temperature of 230°C and a pressure of 3 hPa. The amount of stripping steam amounted to 1 % (w/w) and was supplied over a period of 60 minutes. The stripping process reduced the FFA level from 4.4% in the acid refined sample to only 0.26% (expressed as oleic acid). Accordingly, the preparation of the crude jatropha oil sample provided a raw material that is suitable for transesterification according to the process of the invention.

### EXAMPLE 2

This example illustrates the preparation of a crude palm oil sample by the dry degumming process followed by de-acidification by vacuum stripping. The crude palm oil had an FFA content of 2.07 wt% (expressed as oleic acid) and a phosphorus content of 4.5 ppm. Accordingly, phosphorus removal was not required to prepare the sample for conversion into FAME by transesterification, but bleaching of the sample which contained 438 ppm β-carotene was considered to be desirable. Consequently, the dry degumming process which comprises the use of bleaching earth was chosen as means of preparation. It comprises the following steps:
• The oil is heated to 85°C;
• An amount of 0.02% (w/w) of phosphoric acid of 85% strength is added to the oil and finely dispersed by using a high shear mixer for 1 minute;
• An amount of 0.5% (w/w) of distilled water is added and mixed into the acidified oil under gentle agitation;
• Bleaching earth (Tonsil Optimum 210 FF, commercially available from Sud-Chemie, Munich, Germany) is added in an amount of 1.5 % (w/w) and dispersed into the oil;
• The earth is allowed to act over a period of 30 minutes while the temperature is raised to 95°C and a vacuum of 50 hPa is maintained;
• Finally the sample is filtered using a Whatman No. 1 filter paper in a pre-heated Buchner filter.
The dry degumming process reduced the phosphorus content of the palm oil sample from 4.5 ppm to 0.4 ppm and the iron content from 1.6 ppm to less than 50 ppb. It also reduced the β-carotene content.

The dry degummed sample was subjected to a vacuum stripping process at 280°C and 3 hPa for a period of 50 minutes while supplying 1 % of steam. this reduced the FFA content of the degummed oil to 0.018 wt% (expressed as oleic acid). Accordingly, the dry degumming process followed by the stream stripping process provided a material suitable for being converted into FAME by the process according to the invention.

### EXAMPLE 3

This example illustrates the effect of scrubbing vapours in successive stages. For this purpose, a continuous vacuum stripping unit used for the physical refining of vegetable oil at a throughput of some 40 tonnes per hour was provided with two scrubbers in series. The unit comprised a packed column at the top for the removal of the main amount of FFA and underneath, a number of steam-sparged trays ensured proper deodorisation of the oil.

The soya bean oil used in this example had an FFA content of 1.5 wt% (expressed as oleic acid), a tocopherols content of 600 ppm, and a total sterol content of 4,000 ppm. However, some of the sterol esterified during the high temperature steam stripping process with free fatty acids present. This decreases their volatility and prevents their being removed from the oil being vacuum stripped. The content of non-esterified, free sterols of the soya bean oil amounted to 3,000 ppm.

The oil to be vacuum stripped was heated to 260°C before being divided over the packed column. An amount of 5 kg steam per tonne of oil was fed into the column from underneath. The pressure maintained by the vacuum system at the top of the column was 2 hPa and the pressure drop over the column was 1 hPa. Because of the evaporation of FFA and further heat losses, the oil temperature dropped by 3°C when passing through the column.

In the deodorisation trays the oil was sparged with a total of 5 kg steam per tonne of oil and the vapours leaving the trays and the column were all collected and passed through the two scrubbers. The condensate in the first scrubber was maintained at a temperature of 140°C and thus aimed at condensing the tocopherols and the sterols. The condensate circulating over the second scrubber was kept at 50°C, being the lowest temperature that does not cause solidification.

In the first scrubber, which was kept at 140°C, the distillate was collected at a rate of 300 kg per hour and contained 65 wt% FFA, 11 wt% sterols and 4 wt% tocopherols. The physically refined soya bean oil had an FFA content of 0.07 wt% (expressed as oleic acid) and still contained 365 ppm of tocopherols and 2,200 ppm of free sterols.

The second scrubber kept at 50°C collected distillate at a rate of some 400 kg per hour and this distillate consisted of more than 98% by weight of FFA. It therefore constitutes an ideal raw material for certain embodiments of the process according to the invention.

### EXAMPLE 4

The acid refined and deacidified jatropha oil prepared in Example 1 was transesterified with methanol by a process comprising the following steps:
• An amount of approximately 250 g of the pre-treated oil is heated to a temperature of 60°C;
• An amount of 46.8 g of anhydrous methanol and 5 g of a 30% (w/w) solution of sodium methanolate in methanol are added. On a molar basis, these amounts correspond to about 100% excess;
• The mixture is then allowed to react for 2 hours at 60°C while being gently agitated;
• After this period of time, the reaction mixture is transferred to a separation funnel and the lower, alcoholic layer is drawn off;
• An amount of 3 ml of an 3% (w/w) citric acid solution is added, mixed by shaking the separation funnel, allowed to separate, and drawn off;
• The remaining FAME are washed several times with hot water until neutral and finally dried at 120°C and 50 hPa.

The purity of the resulting FAME was assessed by GLC showing an FFA content of only 0.08%, a monoglyceride content on 0.12% whereas diglycerides and triglycerides could not be detected. The purity was therefore 99.8%. The phosphorus content was found to amount to only 0.6 ppm. This illustrates that a fatty feed containing appreciable amounts of phosphorus (23.2 ppm) can yield FAME with far less phosphorus (0.6 ppm) and thus demonstrates that complete degumming of the fatty feed to a low, e.g. less than 10 ppm or even less than 5 ppm, phosphorus content is not necessary to attain a low residual phosphorus content of the biodiesel produced by the process according to the invention.

On an industrial scale, a smaller excess of methanol suffices, especially if this methanol is not added all at once but in stages and after the glycerol resulting from the previous stage has been removed. Accordingly, a fatty feed that may comprise an esterification product in accordance with the embodiment shown in FIG. 3, can be transesterified using an almost stoichiometric amount of methanol to an extent of some 75%. After phase separation, a further amount of methanol containing some sodium methanolate is added which amount corresponds to about 50% of the theoretical stoichiometric requirement. Transesterification with this amount increases the conversion to more than 98%.

If then after phase separation, a third amount of methanol containing sodium methanolate is added to the FAME phase, the conversion can be increased to more than 99.9% and besides, the resulting alcoholic phase can be recycled to the first stage since the catalyst is still quite active. Accordingly this multi-stage process not only reduces the amount of methanol involved and thus the excess that has to be removed by evaporation and the concomitant energy requirement of the process, but it also increases the conversion and thus the biodiesel yield.

### EXAMPLE 5

In this example, the esterification of a stream rich in FFA with methanol will be illustrated for both the batch process and the continuous process. The FFA rich stream originated from the physical refining of palm oil. In the batch process, an amount of 500 g of a feedstock with an FFA content of 82.9 % (expressed as oleic acid) was mixed with 530 g of methanol containing 3.3 g sulphuric acid (98% strength), corresponding to 0.8 g per 100 g FFA. The temperature of the mixture was brought to 80°C and the mixture was agitated with a magnetic stirrer for 60 minutes. After that reaction time, the agitator was stopped and the reaction mixture was allowed to separate into two phases. The upper phase containing the excess methanol, water and sulphuric acid weighed 475 g and the lower fatty phase weighed 557 g. Its acid value was found to be only 3.14 mg KOH/g corresponding to some 1.58% or 7.9 g FFA. By assuming a density of the reaction mixture of 875 kg/m³, the average esterification rate can be calculated as 345 g per litre per hour.

In a continuous process, a feedstock with an FFA content of 84.5 % (expressed as oleic acid) was mixed with 1 g concentrated (98%) sulphuric acid per gram of FFA and 0.9 parts of methanol per part of feedstock. The mixture was pumped via a heat exchanger through two tubular reactors in series that had a combined volume of 6.07 litres. At a feed rate of 7.23 1/h, a reactor inlet temperature of 122°C and an outlet temperature of 124°C, the operating pressure was 12.5 bar. The residual FFA content of the fatty phase was 2.5%. This corresponds to an average rate of esterification of some 440 g FFA per litre per hour, which is an even higher rate than observed in the batch process.

In both processes, the residual FFA content was quite low which means that the initial rates of esterification were considerably higher than the average rates. In Industrial practice, a compromise will have to be found between the average rate of esterification and the residual FFA content.

### EXAMPLE 6

In the process according to the invention, the FFA rich stream can also be esterified with glycerol. Accordingly, variable amounts of glycerol were added to a waste fat stream with an FFA content of 85%. The mixture was introduced into a Rotavapor flask that was evacuated to a pressure of 50 mbar absolute, and heated by an oil bath. Table 1 below shows the values of various process parameters of this embodiment of the invention.

**Table 1**

| | | | | | | | | |
|---|---|---|---|---|---|---|---|---|
| glycerol (g/100 g) | 15 | 30 | 75 | 30 | 15 | 30 | 30 | 30 |
| temperature (°C) | 150 | 190 | 190 | 190 | 150 | 190 | 190 | 190 |
| reaction time (min.) | 60 | 240 | 180 | 300 | 120 | 60 | 120 | 180 |
| catalyst type | --- | --- | --- | --- | *p*TSA | *p*TSA | *p*TSA | *p*TSA |
| catalyst amount (%) | | | | | 1 | 1 | 1 | 1 |
| residual FFA (%) | 64.0 | 5.5 | 7.6 | 2.7 | 30.0 | 20.7 | 8.5 | 5.0 |
| monoglycerides | 10.2 | 14.0 | 18.6 | 12.0 | NA | 11.3 | 6.9 | 5.6 |
| Diglycerides | 3.9 | 24.0 | 20.7 | 24.1 | NA | 9.9 | 20.5 | 20.3 |
| Triglycerides | 4.4 | 5.8 | 5.5 | 12.0 | NA | 3.3 | 8.7 | 14.9 |

| | | | | | | | | |
|---|---|---|---|---|---|---|---|---|
| *p*TSA = para toluene sulphonic acid NA = not analysed Table 1 shows that low residual FFA contents can be attained and are favoured by an excess of glycerol, an increase in temperature and the presence of a catalyst such as p-toluene sulphonic acid. The table also shows that esterification can be carried out in the presence of substantial amounts of unknown compounds, the presence of which is demonstrated by the fact that the sum of the FFA and the glycerides is lower than 100%. Their concentration was determined by GLC and using betuline as internal standard. | | | | | | | | |

Accordingly, this example illustrates that the use of high-acidity fatty feeds in the process according to the invention also comprising unknown compounds can be profitably executed by esterifying said FFA with glycerol and then subjecting the esterification product to a vacuum stripping process that removes said unknown compounds. This course of events provides a purified fatty feed to the transesterification step and ensures the robust operation of said process.

The present invention also includes process equipment for the production of esters of fatty acids and C₁-C₅ alkyl alcohols. The process equipment may, for example, include dedicated equipment or existing equipment customised to carry out the functions of the present invention. The equipment comprises:
Means for neutralising a fatty feed comprising a triglyceride oil or fat, partial glycerides and/or free fatty acids by vacuum stripping at a temperature from 200°C to 280°C, thus providing a vapour stream and a residue,
Means for collecting a distillate by scrubbing said vapour stream,
Means for transesterifying said residue with a C₁-C₅ alkyl alcohol while using an alkaline catalyst, and
Means for separating the transesterification reaction mixture into a fraction comprising C₁-C₅ alkyl esters of fatty acids and an alcoholic fraction.

The invention is well adapted to carry out the objects and attain the ends and advantages mentioned, as well as others inherent therein. While the invention has been depicted, described and defined by reference to exemplary embodiments of the invention, such references do not imply a limitation on the invention, and no such limitation is to be inferred. The invention is capable of considerable modification, alteration, and equivalence in form and function, as will occur to those ordinarily skilled in the pertinent arts and having the benefit of this disclosure. The depicted and described embodiments of the invention are exemplary only, and are not exhaustive of the scope of the invention. It is intended that all such variations within the scope of the invention, giving full cognisance to equivalence in all respects, be included within the scope of the appended claims.

## Claims

1. Process for the production of esters of fatty acids and C₁-C₅ alkyl alcohols comprising the steps of:
(a) neutralising a fatty feed comprising a triglyceride oil or fat, partial glycerides and/or free fatty acids by vacuum stripping at a temperature from 200°C to 280°C, thus providing a vapour stream and a residue,
(b) collecting a distillate by scrubbing said vapour stream,
(c) transesterifying said residue with a C₁-C₅ alkyl alcohol while using an alkaline catalyst,
(d) separating the transesterification reaction mixture from step (c) into a fraction comprising C₁-C₅ alkyl esters of fatty acids and an alcoholic fraction.

2. Process according to claim 1, in which the fatty feed has been subjected to acid refining before being neutralised in step (a).

3. Process according to claim 1, in which the fatty feed has been subjected to dry degumming before being neutralised in step (a).

4. Process according to any of claims 1 to 3, in which the phosphorus content of the fatty feed before step (a) is less than 25 ppm.

5. Process according to claim 2 or claim 3, wherein the phosphorus content of the fatty feed after said acid refining or dry degumming step is less than 10 ppm.

6. Process according to any of claims 1 to 5, in which the vapour stream from the vacuum stripping process of step (a) are condensed in two or more successive stages.

7. Process according to any of claims 1 to 6, comprising the further step of washing the fraction comprising C₁-C₅ alkyl esters of fatty acids obtained in step (d) with acidified water.

8. Process according to any of claims 1 to 7, comprising the further step of:
(e) esterifying a stream rich in fatty acids with an alcohol while using an acid catalyst.

9. Process according to claim 8, in which said stream rich in fatty acids comprises part of the distillate collected in step (b).

10. Process according to claim 8, in which said stream rich in fatty acids comprises acid oils resulting from the acidulation of a soap stock.

11. Process according to claim 8, in which said stream rich in fatty acids comprises a hydrolysate of gums, acid oils or soap stock.

12. Process according to any of claims 8 to 11, in which the alcohol used in step (e) is a C₁-C₅ alkyl alcohol.

13. Process according to any of claims 8 to 11, in which the alcohol used in step (e) is glycerol.

14. Process according to claim 12 or claim 13, in which the esterification product resulting from step (e) is mixed with the neutralised fatty feed from step (a).

15. Process according to claim 13, in which the esterification product resulting from step (e) is mixed with the fatty feed before the latter is neutralised in step (a).

16. Process according to any of claims 1 to 15, in which the alcoholic fraction resulting from step (d) is acidulated and the fatty fraction formed is isolated.

17. Process according to claim 16, **characterised** that the isolated fatty fraction is mixed with the oil to be neutralised in step (a).

18. Process equipment for the production of esters of fatty acids and C₁-C₅ alkyl alcohols comprising:
(a) Means for neutralising a fatty feed comprising a triglyceride oil or fat, partial glycerides and/or free fatty acids by vacuum stripping at a temperature from 200°C to 280°C, thus providing a vapour stream and a residue,
(b) Means for collecting a distillate by scrubbing said vapour stream,
(c) Means for transesterifying said residue with a C₁-C₅ alkyl alcohol while using an alkaline catalyst, and
(d) Means for separating the transesterification reaction mixture into a fraction comprising C₁-C₅ alkyl esters of fatty acids and an alcoholic fraction.
